# EUROPEAN PATENT SPECIFICATION

(11) **EP 0 807 075 B1**
(45) Date of publication and mention of the grant of the patent: **18.08.1999**
(21) Application number: 96906252.0
(22) Date of filing: 01.02.1996
(51) Int. Cl.: B65D 75/58, B65D 75/46

(54) **A FLEXIBLE TUBULAR PACKAGE AND PACKAGED TAMPON APPLICATOR**
SCHLAUCHPACKUNG SOWIE VERPACKTE TAMPONEINFÜHRVORRICHTUNG
EMBALLAGE TUBULAIRE SOUPLE ET APPLICATEUR DE TAMPON EMBALLE

(30) Priority: 01.02.1995 US 382321
(43) Date of publication of application: 19.11.1997
(73) Proprietor: McNEIL-PPC, INC., Skillman, New Jersey 08558 (US)
(72) Inventor: ENGQVIST, Helena, Princeton, NJ 08540 (US); ISKRA, Michael, Bridgewater, NJ 08807 (US); SCHOELLING, Hans, Werner, D-58256 Ennepetal (DE); SMAGA, Rainer, D-44879 Bochum (DE)
(74) Representative: Mercer, Christopher Paul
(86) International application number: US9601363
(87) International publication number: WO9623711

(56) References cited:
- WO-A-93/11054
- DE-U- 8 606 170
- US-A- 4 648 513
- US-A- 4 881 644
- US-A- 5 133 457

## Description

### Field of the Invention

The present invention relates generally to a flexible, elongate overwrap package having a generally tubular configuration with flattened ends and a longitudinal seam useful as a reinforced tear strip for opening the package. The overwrap can be used to protect sanitary protection products such as tampons during storage, before use.

### Background of the Invention

Packages for tubular products are known in the art. In applications where it is important to protect the enclosed product from contamination due to inadvertent opening, such as tampon overwraps, the package is formed of materials which are relatively strong. Such packages are often formed of plastic films, paper, and coated paper. The use of strong materials increases the difficulty which a user faces in opening the package in a controlled manner and at a controlled location.

Attempts to provide overwraps formed of materials having relatively high tensile, burst, and tear strengths while nonetheless allowing for controlled opening of the package include Kadel, U.S. Patent No. 5,133,457, and Norquest et al., U.S. Patent No. 4,881,644. These packages utilize materials such as voided polypropylene film, low density polyethylene, and paper. The wrappers described in these references include wrap separating means such as score lines which run perpendicular to the length dimension of the package. The use of score lines to preferentially locate overwrap openings has several drawbacks. First, forming score lines requires a high degree of process control to avoid forming undesirable perforations in the overwrap material. Such perforation s can be a source for contamination. Second, the formation of score lines in the package requires additional process steps beyond those necessary to form the package itself.

US- A- 4 648 513 discloses one such package with perforations. This package serves a dual purpose by being formed of a pouch with a sealable flap. The pouch is initially wrapped as a sheet around the product and sealed at each end. Perforations allow the package to be opened , and subsequently the package may be reused as a pouch with sealable flap.

Therefore, what is needed is a simple, strong overwrap package which has a generally tubular shape and which incorporates features to help the user to open the package in a controlled manner at a controlled location.

### Summary of the Invention

The present invention relates to a flexible, overwrap package formed of a generally tubular sheet of packaging material having an outer surface and a sealable inner surface. The packaging material is formed into a tube and sealed along a longitudinal seam and first and second generally flattened ends. The sealed package has a first lateral side. The longitudinal seam is oriented proximate the first lateral side of the first and second generally flattened ends. The seam has (a) a first narrow longitudinal strip portion of outwardly facing, sealable inner surface which is superposed over a corresponding longitudinal strip portion of the outer surface, (b) a second narrow longitudinal strip portion of outwardly facing outer surface adjacent said sealable inner surface, and (c) a third narrow longitudinal strip portion of inwardly facing sealable inner surface which is superposed over and sealed to the first and second longitudinal strip portions. Preferably, the first narrow strip is formed by folding a portion of the inner surface of the packaging material back over the outer surface. The seam therefore, provides a reinforced tearing strip, and a tear originating at the first generally flattened end proximate the longitudinal seam away from the first lateral side is capable of following the longitudinal seam toward the second generally flattened end.

The present invention also provides a packaged tampon applicator as defined in claim 10.

### Brief Description of the Drawing

Figure 1 is a perspective view of a tampon overwrap according to the present invention.
Figure 2 is a top plan view of the tampon overwrap.
Figure 3 is a side elevation of the tampon overwrap.
Figure 4 is an end elevation of the tampon overwrap.
Figures 5A and 5B are partial schematic representations of the process of making the package of the present invention.
Figure 5C is top view of an additional schematic representation of the process of making the package of the present invention.

### Detailed Description of the Invention

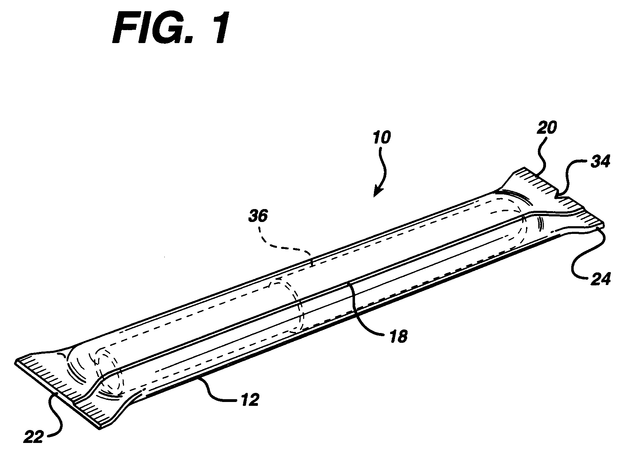

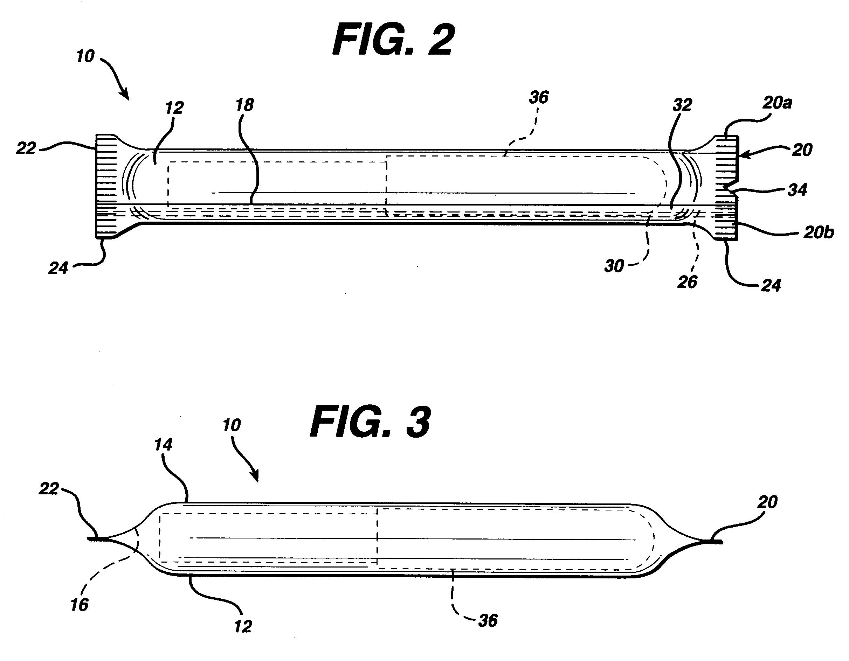

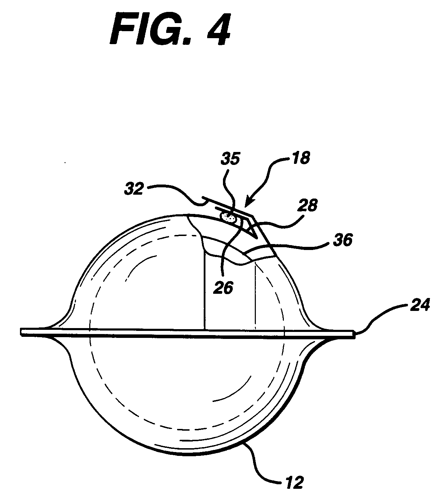

Referring to Figs. 1-4, a flexible, overwrap package 10 is formed of a generally tubular sheet of packaging material 12 having an outer surface 14, a sealable inner surface 16, a longitudinal seam 18, and first and second generally flattened ends 20 and 22, respectively, having a first lateral side 24. The longitudinal seam 18 is oriented proximate the first lateral side 24 of the first and second generally flattened ends 20, 22.

The seam 18 has a first narrow longitudinal strip portion of outwardly facing, sealable inner surface 26. This strip portion 26 is superposed over a corresponding longitudinal strip portion of the outer surface 28. The seam 18 also includes a second narrow longitudinal strip portion of outwardly facing outer surface 30 adjacent said sealable inner surface 26. Another portion of the seam 18 is a third narrow longitudinal strip portion of inwardly facing sealable inner surface 32 which is superposed over and sealed to the first and second longitudinal strip portions 26, 30. Preferably, the first narrow strip 26 is formed by folding a portion of the inner surface of the packaging material back over the outer surface 28. The seam 18 therefore, provides a reinforced tearing strip, and a tear originating at the first, generally flattened end 20 proximate the longitudinal seam 18 away from the first lateral side 24 is capable of following the longitudinal seam 18 toward the second, generally flattened end 22. In order to locate the beginning of the tear, a notch 34 can be formed in the first, generally flattened end 20.

In an alternative embodiment, the longitudinal seam 18 may also include reinforcing means 35. The reinforcing means 35 is preferably an element which is continuous along the length of the longitudinal seam 18. A representative, non-limiting list of useful reinforcing means 18, includes string, plastic tape, monofilament, a continuous line of a polymeric mass such as hot melt, cold glue, and the like.

In use, a consumer can grip the left side 20a of the first end 20 between the thumb and forefinger of the user's first hand, and the right side 20b of the first end 20 between the thumb and forefinger of the user's second hand. The user's second hand can then gently twist and/or tear the right side of the overwrap package 10 away from the left side, and a tear originated at the notch 34 will generally follow the seam 18 to expose the contents 36 of the overwrap package 10.

While the overwrap package 10 is illustrated as protecting an applicator tampon 36, it can also be used to protect relatively long objects such as digital tampons, tampon inserters, vaginal medication and/or spermicide applicators, feminine hygiene products, and the like.

The packaging material can be any suitable sheet-like material including, without limitation, polymeric films, paper, woven and nonwoven fabrics, any combination thereof, and the like. These sheet-like materials may be coated and/or laminated, as desired. Preferably, the packaging material is a polymeric film, paper or coated paper. More preferably, the packaging material has at least one sealable surface, e.g., a thermoplastic layer or a cohesive sealing layer, for mechanical sealing, heat sealing, ultrasonic sealing, cold sealing, or any combination thereof, and the like. In a particularly preferred embodiment, the packaging material is a polymer-coated paper. In a more preferred embodiment, the packaging material is polyethylene-coated paper. Such a coated paper is low density polyethylene-coated, white kraft paper having a basis weight of 55 g/m² (30 g/m² paper and 25 g/m² coating). This material is available as SUPRAFERM 67030-25 from PKL, of Linnich, Germany.

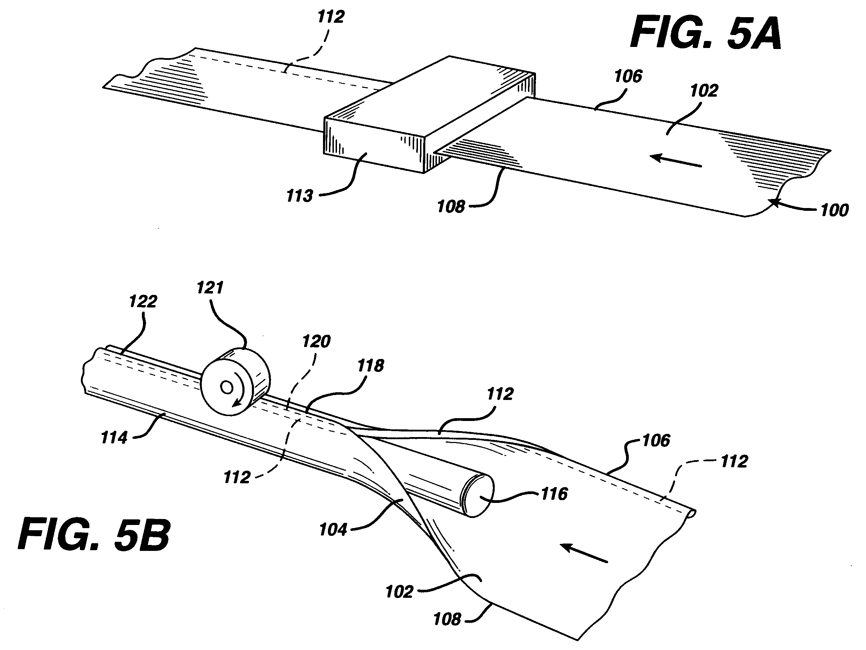

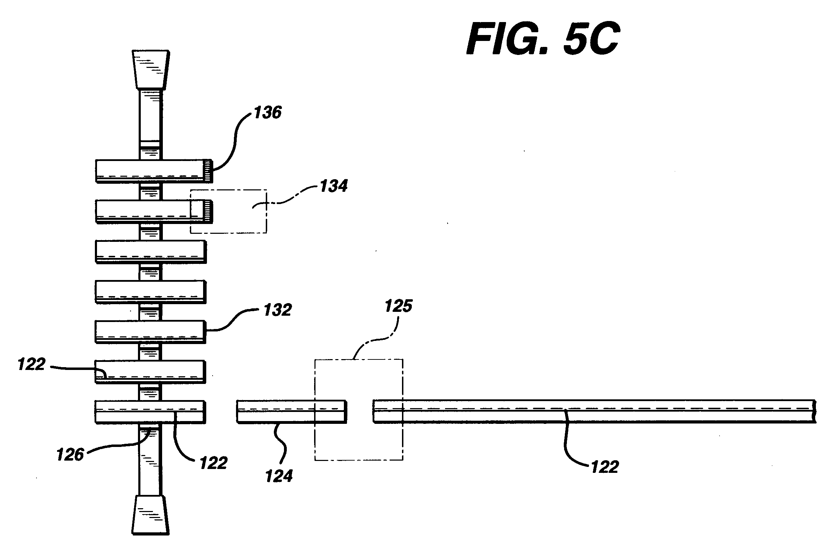

Referring now to Figs. 5A-5C, the overwrap package can be formed by conveying a sheet-like ribbon of flexible packaging material 100 in a machine direction corresponding to the longitudinal axis of the packaging material 100. The ribbon of packaging material 100 has a sealable surface 102 and an outer surface 104, first and second lateral edges 106 and 108, respectively, and a longitudinal axis. A first sealing strip 112 can be formed by folding a narrow portion of the packaging material 100 adjacent the first lateral edge 106 approximately 180° back at a folding station 113.

The folded packaging material 100 can be formed into a tube 114 by wrapping the material 100 around a forming mandrel 116 which has a diameter corresponding to the inside diameter of the finished tubular overwrap package. The packaging material 100 is wrapped around the mandrel 116 so the sealable surface 102 forms the inside surface of the tube 114, the outer surface 104 forms the outside surface of the tube 114, and a sealable strip 118 adjacent the second lateral edge 108 overlaps the first sealing strip 112 and a second sealing strip 120 which is formed of the outer surface 104 which is disposed adjacent to the first sealing strip 112, away from the first lateral edge 106. The sealable strip 118 adjacent the second lateral edge 108 can then be sealed to the first and second sealing strips 112, 120, at a sealing station 121 to form a longitudinal seam 122, by means known to those of ordinary skill in the art. For example, the surfaces can be heat sealed, ultrasonically sealed, adhesively sealed, cold sealed, mechanically sealed, cohesively sealed, and the like.

The sealed tube 114 can then be separated at a separation station 125 to form individual package stock 124. This operation can be performed with cutting blades, rotary knife, shear cut, crush cut, water jet, straining preweakened areas, laser, and other separation operations known to those skilled in the art. The individual package stock 124 can be transferred onto carriers 126 moving transversely to the continuous tube. The timing of the transfer of the individual package stock 124 into the carriers 126 orients the longitudinal seam 122 to a position corresponding to a location proximate a lateral edge of the flattened ends when the ends are sealed. The orienting means 130 can orient the longitudinal seam 122 by rotating the package stock 124 about 90°. The degree of rotation is not critical, and it can range from about 30° to about 150°. Thus, the rotation of the package stock 124 is sufficient to locate the seam 122 proximate the lateral edge of the flattened ends when sealed. The oriented package stock 132 can then be transferred to a first end sealing station 134, e.g., heated sealing wheels, rollers, and/or belts, which flattens and seals a first longitudinal end 136 of the oriented package stock 132. The flattened first longitudinal end 136 can also be notched. The overwrap package can then be filled with product and the second longitudinal end can be flattened and sealed. These steps are well known to those of ordinary skill in the art.

The specification and embodiments above are presented to aid in the complete and non-limiting understanding of the invention disclosed herein. Since many variations and embodiments of the invention can be made without departing from its scope, the invention resides in the claims hereinafter appended.

## Claims

1. A flexible, overwrap package (10) comprising a generally tubular sheet of packaging material (12) having an outer surface (14), a sealable inner surface (16), a longitudinal seam (18), and first and second generally flattened ends (20,22) having a first lateral side (24), the longitudinal seam (18) comprising:
a) a first narrow longitudinal strip portion of outwardly facing, sealable inner surface (26) which is superposed over a corresponding longitudinal strip portion of the outer surface (28);
characterised in that the longitudinal seam (18) is oriented proximate the first lateral side (24) of the first and second generally flattened ends (20,22) and further comprises:
b) a second narrow longitudinal strip portion of outwardly facing outer surface (30) adjacent said sealable inner surface (26);
c) a third narrow longitudinal strip portion of inwardly facing sealable inner surface (32) which is superposed over and sealed to the first and second longitudinal strip portions (26,30);
whereby a tear originating at the first generally flattened end (20) proximate the longitudinal seam (18) away from the first lateral side (24) is capable of following the longitudinal seam (18) toward the second generally flattened end (22).

2. The overwrap package (10) of claim 1, wherein the sealable inner surface (16) is a heat sealable surface.

3. The overwrap package (10) of claim 1, wherein the longitudinal seam (18) further comprises reinforcing means (35).

4. The overwrap package (10) of claim 3, wherein the reinforcing means (35) located between the third narrow strip portion and at least one of the first and second narrow strip portions of the longitudinal seam (18).

5. The overwrap package (10) of claim 3, wherein the reinforcing means (35) is selected from the group consisting of string, polymeric tape, and hot melt.

6. The overwrap package (10) of claim 1, further comprising a notch (34) located at the first generally flattened end (20) whereby a tear can originate at a controlled location.

7. The overwarp package (10) of claim 1, wherein the packaging material (12) is selected from the group consisting of polymeric films, paper, and coated paper.

8. The overwrap package (10) of claim 7, wherein the packaging material (12) is coated paper.

9. The overwrap package (10) of claim 7, wherein the coated paper comprises a layer of paper and a layer of a polymeric coating.

10. A packaged tampon applicator (36) comprising:
a) a tampon applicator (36) having a barrel member containing a tampon and a plunger member which is configured for telescopically sliding within the barrel member and expelling the tampon from the barrel;
b) an overwrap package (10) comprising a generally tubular sheet of packaging material (12) having an outer surface (14), a sealable inner surface (16), and first and second generally flattened ends (20,22) having a first lateral side (24), characterised in that the overwrap package comprises a longitudinal seam (18) oriented proximate the first lateral side (24) of the first and second generally flattened ends (20,22) the longitudinal seam (18) comprising:
i) a first narrow longitudinal strip portion of outwardly facing, sealable inner surface (26) which is superposed over a corresponding longitudinal strip portion of the outer surface (28);
ii) a second narrow longitudinal strip portion of outwardly facing outer surface (30) adjacent said sealable inner surface (26);
iii) a third narrow longitudinal strip portion of inwardly facing sealable inner surface (32) which is superposed over and sealed to the first and second longitudinal strip portions (26,30);
whereby a tear originating at the first generally flattened end (24) proximate the longitudinal seam (18) away from the first lateral side (24) is capable of following the longitudinal seam (18) toward the second generaly flattened end (20).

## Patentansprüche

1. Flexible Einschlag-Verpackung (10), die eine im allgemeinen rohrförmige Lage (12) aus einem Verpackungsmaterial mit einer Außenfläche (14), einer abdichtbaren Innenfläche (16), einem längs-verlaufenden Saum (18) und einem ersten sowie zweiten abgeflachten Ende (20,22) mit einer ersten Querseite (24) umfaßt, wobei der längsverlaufende Saum (18) folgendes umfaßt:
a) einen ersten schmalen längsverlaufenden Streifenabschnitt der außenliegenden, abdichtbaren Innenfläche (26), der über einen entsprechenden längsverlaufenden Streifenabschnitt der Außenfläche (28) gelegt ist;
dadurch gekennzeichnet, daß der längsverlaufende Saum (18) in Nähe zur ersten Querseite (24) des ersten und zweiten im allgemeinen abgeflachten Endes (20,22) ausgerichtet ist und weiter folgendes umfaßt:
b) einen zweiten schmalen längsverlaufenden Streifenabschnitt der außenliegenden Außenfläche (30), benachbart zu der abdichtbaren Innenfläche (26);
c) einen dritten schmalen längsverlaufenden Streifenabschnitt einer innenliegenden abdichtbaren Innenfläche (32), der über den ersten und zweiten längsverlaufenden Streifenabschnitt (26,30) gelegt und mit denselben abgedichtet ist;
wodurch ein Riß, der an dem ersten im allgemeinen abgeflachten Ende (20) unmittelbar zu dem längsverlaufenden Saum (18) benachbart, von der ersten Querseite (24) weg entsteht, dazu fähig ist, dem längsverlaufenden Saum (18) zu dem zweiten im allgemeinen abgeflachten Ende (22) hin zu folgen.

2. Einschlag-Verpackung (10) nach Anspruch 1, dadurch gekennzeichnet, daß die abdichtbare Innenfläche (16) eine heißdichtbare Fläche ist.

3. Einschlag-Verpackung (10) nach Anspruch 1, dadurch gekennzeichnet, daß der längsverlaufende Saum (18) ferner ein Verstärkungsmittel (35) umfaßt.

4. Einschlag-Verpackung (10) nach Anspruch 3, dadurch gekennzeichnet, daß das Verstärkungsmittel (35) zwischen dem dritten schmalen Streifenabschnitt und dem ersten und/oder zweiten schmalen Streifenabschnitt des längsverlaufenden Saums (18) gelegen ist.

5. Einschlag-Verpackung (10) nach Anspruch 3, dadurch gekennzeichnet, daß das Verstärkungsmittel (35) aus der aus Schnurband, polymerem Band und Heißschmelze bestehenden Gruppe ausgewählt ist.

6. Einschlag-Verpackung (10) nach Anspruch 1, gekennzeichnet durch einen an dem ersten im allgemeinen abgeflachten Ende (20) gelegenen Einschnitt (34) umfaßt, wodurch ein Riß an einem kontrollierten Ort entstehen kann.

7. Einschlag-Verpackung (10) nach Anspruch 1, dadurch gekennzeichnet, daß das Verpackungsmaterial (12) aus der aus polymerem Film, Papier und beschichtetem Papier bestehenden Gruppe ausgewählt ist.

8. Einschlag-Verpackung (10) nach Anspruch 7, dadurch gekennzeichnet, daß das Verpackungsmaterial (12) beschichtetes Papier ist.

9. Einschlag-Verpackung (10) nach Anspruch 7, dadurch gekennzeichnet, daß das beschichtete Papier eine Papierlage und eine polymere Beschichtungslage umfaßt.

10. Verpackte Tamponeinführvorrichtung (36), mit:
a) einer Tamponeinführvorrichtung (36) mit einem ein Tampon enthaltenden Hülsenteil und einem Stösselteil, das zum teleskopartigen Gleiten in dem Hülsenteil und zum Austreiben des Tampons aus der Hülse ausgestaltet ist;
b) einer Einschlag-Verpackung (10), die eine im allgemeinen rohrförmige Lage (12) aus einem Verpackungsmaterial mit einer Außenfläche (14), einer abdichtbaren Innenfläche (16) und einem ersten und zweiten im allgemeinen abgeflachten Ende (20,22) mit einer ersten Querseite (24) umfaßt, dadurch gekennzeichnet, daß die Einschlag-Verpackung einen in Nähe zur ersten Querseite (24) des ersten und zweiten im allgemeinen abgeflachten Endes (20,22) längsverlaufenden Saum (18) umfaßt, mit:
i) einem ersten schmalen längsverlaufenden Streifenabschnitt der außenliegenden, abdichtbaren Innenfläche (26), der über einen entsprechenden längsverlaufenden Streifenabschnitt der Außenfläche (28) gelegt ist;
ii) einem zweiten schmalen längsverlaufenden Streifenabschnitt der außenliegenden Außenfläche (30), benachbart zu der abdichtbaren Innenfläche (26);
iii) einem dritten schmalen längsverlaufenden Streifenabschnitt einer innenliegenden abdichtbaren Innenfläche (32), der über den ersten und zweiten längsverlaufenden Streifenabschnitt (26,30) gelegt und mit denselben abgedichtet ist;
wodurch ein Riß, der an dem ersten im allgemeinen abgeflachten Ende (24) unmittelbar zu dem längsverlaufendenden Saum (18) benachbart, von der ersten Querseite (24) weg entsteht, dazu fähig ist, dem längsverlaufenden Saum (18) zu dem zweiten im allgemeinen abgeflachten Ende (20) hin zu folgen.

## Revendications

1. Un emballage de survenveloppement (10) flexible, comprenant une feuille globalement tubulaire en matériau d'emballage (12), ayant une surface extérieure (14), une surface intérieure (16) scellable, une ligne de soudage (18) longitudinale, et des première et deuxième extrémités (20,22) globalement aplaties ayant une première face latérale (24), la ligne de soudage (18) longitudinale comprenant :
a) une première partie de bande longitudinale étroite, d'une surface intérieure (26) scellable tournée vers l'extérieur, placée sur une partie de bande longitudinale correspondante de la surface extérieure (28) ;
caractérisé en ce que la ligne de soudage (18) longitudinale est orientée à proximité de la première face latérale (24) des première et deuxième extrémités (20, 22) globalement aplaties et comprend en outre :
b) une deuxième partie de bande longitudinale étroite d'une surface extérieure (30) tournée vers l'extérieur, adjacente à ladite surface intérieure (26) scellable ;
c) une troisième partie de bande longitudinale étroite d'une surface intérieure (32) scellable tournée vers l'intérieur, placée sur et scellée aux première et deuxième parties de bande longitudinale (26,30) ;
de manière qu'un déchirement, prenant origine à la première extrémité (20) globalement aplatie, à proximité de la ligne de soudage (18) longitudinale, en s'écartant de la première face latérale (24) soit en mesure de suivre la ligne de soudage (18) longitudinale en direction de la deuxième extrémité (22) globalement aplatie.

2. L'emballage de surenveloppement (10) selon la revendication 1, dans lequel la surface intérieure (16) scellable est une surface thermo-scellable.

3. L'emballage de surenveloppement (10) selon la revendication 1, dans lequel la ligne de soudage (18) longitudinale comprend en outre des moyens de renforcement (35).

4. L'emballage de surenveloppement (10) selon la revendication 3, dans lequel les moyens de renforcement (35) sont placés entre la troisième partie de bande étroite et au moins une des première et deuxième parties de bande étroite de la ligne de soudage (18) longitudinale.

5. L'emballage de surenveloppement (10) selon la revendication 3, dans lequel les moyens de renforcement (35) sont sélectionnés dans le groupe constitué d'un cordon, d'un ruban polymère et d'un produit thermo-fusible.

6. L'emballage de surenveloppement (10) selon la revendication 1, comprenant en outre une encoche (34) ménagée sur la première extrémité (20) globalement aplatie, de manière que le déchirement puisse prendre origine en un emplacement contrôlé.

7. L'emballage de surenveloppement (10) selon la revendication 1, dans lequel le matériau d'emballage (12) est sélectionné dans le groupe constitué de films polymères, de papier, et de papier revêtu.

8. L'emballage de surenveloppement (10) selon la revendication 7, dans lequel le matériau d'emballage (12) est du papier revêtu.

9. L'emballage de surenveloppement (10) selon la revendication 7, dans lequel le papier revêtu comprend une couche de papier et une couche de revêtement polymère.

10. Un applicateur de tampon (36) emballé, comprenant :
a) un applicateur de tampon (36) ayant un organe formant cylindre, contenant un tampon, et un organe formant piston, configuré pour glisser de façon télescopique à l'intérieur de l'organe formant cylindre et expulser le tampon du cylindre ;
b) un emballage de surenveloppement (10) comprenant une feuille globalement tubulaire en matériau d'emballage (12) ayant une surface extérieure (14), une surface intérieure (16) scellable, et des première et deuxième extrémités (20, 22) globalement aplaties, ayant une première face latérale (24), caractérisé en ce que l'emballage de surenveloppement comprend une ligne de soudage (18) longitudinale orientée à proximité de la première face latérale (24) des première et deuxième extrémités (20, 22) globalement aplaties, la ligne de soudage (18) longitudinale comprenant :
i) une première partie de bande longitudinale étroite, d'une surface intérieure (26) scellable tournée vers l'extérieur, placée sur une partie de bande longitudinale correspondante de la surface extérieure (28) ;
ii) une deuxième partie de bande longitudinale étroite d'une surface extérieure (30) tournée vers l'extérieur, adjacente à ladite surface intérieure (26) scellable ;
iii) une troisième partie de bande longitudinale étroite d'une surface intérieure (32) scellable tournée vers l'intérieur, placée sur et scellée aux première et deuxième parties de bande longitudinale (26,30) ;
de manière qu'un déchirement, prenant origine à la première extrémité (20) globalement aplatie, à proximité de la ligne de soudage (18) longitudinale, en s'écartant de la première face latérale (24), soit en mesure de suivre la ligne de soudage (18) longitudinale en direction de la deuxième extrémité (22) globalement aplatie.
